(19) Europäisches Patentamt
European Patent Office
Office européen des brevets

(11) **EP 4 755 289 A1**

(12) **EUROPEAN PATENT APPLICATION**

(43) Date of publication:
**10.06.2026 Bulletin 2026/24**

(21) Application number: **24217581.8**

(22) Date of filing: **04.12.2024**

(51) International Patent Classification (IPC):
**A61B 5/00** (2006.01)     **A61B 5/024** (2006.01)

(52) Cooperative Patent Classification (CPC):
**A61B 5/02416; A61B 5/0077; A61B 5/7203**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **EdgeVitals Sàrl
1015 Lausanne (CH)**

(72) Inventors:
• **Vuleti , Miljan
1010 Lausanne (CH)**
• **Papi , Veljko
11010 Belgrade (RS)**
• **Kaempf, Daniel
1023 Crissier (CH)**

(74) Representative: **Weihs, Bruno Konrad
André Roland SA
IP Attorneys & Services
Avenue Collonges 21
1004 Lausanne (CH)**

(54) **REMOTE NONINTRUSIVE HEART RATE DETECTION AND TRACKING FROM VIDEO SIGNALS ON EMBEDDED DEVICES**

(57)     Nonintrusive approaches for vital signs detection and tracking represent appealing methods for improving wellness in broad population ranges and detecting/monitoring health conditions among medical patients. Both industry and academia have shown significant interest in providing reliable contactless reading of vital signs using common video sensors and light modulation properties coming from blood circulation in human skin. Existing approaches propose solutions that can extract the heart rate signal from human faces; they also address the noise challenges from the environment with partial success. We aim to provide accurate heart rate readings from human subjects and human face in situations where temporary in-band noise from subject head motion and lightness changes may occur. Our contribution creates multiple reading channels and brings forth a detection-and tracking method that can efficiently cope with the noise, providing reliable heart-rate readings in stressful conditions. In addition, the method we propose does not rely on sophisticated AI blocks and is, thus, applicable for implementation on embedded and mobile devices with limited processing resources. We demonstrate the claim by implementing and running our method on several embedded and mobile platforms.

FIGURE 2

**Description**

**FIELD OF THE INVENTION**

The present invention relates to a method and corresponding system for obtaining heart rate information from a human using remote photoplethysmography (rPPG).

**BACKGROUND**

Blood volume changes in subcutaneous tissues impact the light absorption and modulate the light reflected from the skin (see for example, Park, Junyung, et al. "Photoplethysmogram analysis and applications: an integrative review." Frontiers in Physiology 12 (2022): 808451, the entire contents of which are incorporated herein by reference). Photoplethysmography (PPG) and remote photoplethysmography (rPPG) methods use this phenomenon to measure the changes in light absorption by the skin and estimate heart rate or other vital signs.

Contact-based photoplethysmography methods emit their own light and illuminate the human skin using light-emitting diodes (LEDs) within close distance ranges. Medical or consumer devices such as oximeters or smart wearable devices implement contact-based measurement and emit their own light. The choice of the emitted light colour maximises the absorption effect.

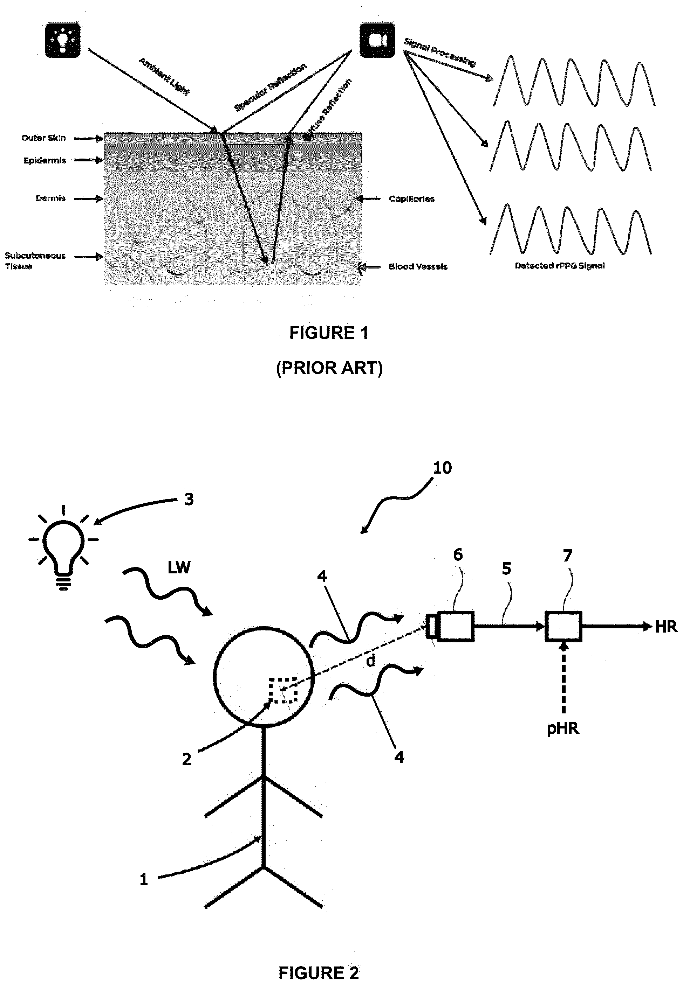

On the other side, remote photoplethysmography (rPPG) relies on light sources-artificial or natural-set farther away from the observed subject. Figure 1 illustrates reflection and absorption of ambient light by human skin as these phenomena are used in rPPG methods to obtain rPPG signals, as known from the prior art. Incoming ambient light penetrates the layers of the skin and underlying tissue, from the outer skin to the epidermis, and subcutaneous tissue, where it is in part absorbed by the blood in blood vessels and capillaries before being reflected according to specular reflection and diffuse reflection. Changes in the properties of the reflected light not perceivable to the human eye may be discerned by sensing the reflected light over time to obtain a time-domain signal or video signal and performing signal processing to obtain remote PPG signals (Detected rPPG signal) which contain information about the blood volume changes in the underlying capillaries and blood vessels.

In remote photoplethysmography, even though the emitted light is not single-coloured as in the contact or close distance range PPG approaches, the effect of the modulation of light reflected from the skin due to blood volume changes is similar, and an appropriate video sensor or camera can detect the blood volume changes from its colour-space channels representing the input signal, or from for example the RGB channels of an acquired video signal, (see for example, Wenjin Wang, Albertus C. den Brinker, Sander Stuijk, and Gerard de Haan. Algorithmic principles of remote PPG. IEEE Transactions on Biomedical Engineering, 64(7):1479-1491, 2017, the entire contents of which are incorporated herein by reference).

Therefore, remote photoplethysmography comprises the use of light reflected from the skin in the form of an acquired video signal (so without skin contact, i.e., remote), to determine physiological processes such as blood volume changes in a blood-perfused subcutaneous tissue portion, wherein these changes are related to vital signs such as heart rate, by detecting subtle, momentary changes in the human subject's skin colour which are not detectable by the human eye.

A nonintrusive method for heart rate detection and tracking must be able to extract reliably the desired vital sign, such as a heart rate, from the input signal, notwithstanding the environmental noise such as signal disturbances coming from light source changes or head movements. These unwanted fluctuations also modulate the captured signal and thus superpose or mask the heart-originated changes.

Below will be discussed related work and state of the art for heart rate detection using rPPG, the contribution of the present invention to this field, and its device and method implementation and porting to target embedded platforms.

Several in-depth surveys list related work relevant for remote vital signs monitoring, see for example McDuff, Daniel. "Camera measurement of physiological vital signs." ACM Computing Surveys 55.9 (2023): 1-40, or Pireh Pirzada, et al., "Remote photoplethysmography (rPPG): A state-of-the-art review." medRxiv, 2023, the entire contents of which are incorporated herein by reference. Other researchers have founded the theoretical bases and models for the growing field of vital signs analysis using rPPG, see for example Wang, Wenjin, et al. "Algorithmic principles of remote PPG." IEEE Transactions on Biomedical Engineering 64.7 (2016): 1479-1491, the entire contents of which are herein incorporated by reference. The related work may be categorised into two major approaches based on: (1) traditional signal processing methods; and (2) deep-learning methods.

In contrast to the traditional methods, deep-learning-based approaches, such as Chen, Weixuan, and Daniel McDuff. "Deepphys: Video-based physiological measurement using convolutional attention networks." Proceedings of the European conference on computer vision (ECCV). 2018, the entire contents of which are herein incorporated by reference, rely entirely on convolutional neural networks (CNNs) to detect heart rate or other physiological signs. The benefits of deep learning approaches come mainly from motion analysis and attention mechanisms. Solving any video-related problem using machine learning takes the advantage of implicit motion modelling, hence it brings the potential of having reliable readings in situations when the observed-subject is moving. Attention mechanisms, on the other side, may leverage from putting more weight on facial features of interest, consequently paying less attention to insignificant image parts. While they offer propitious results for remote vital signs monitoring, most deep learning approaches require increased processing power and memory capacity to store, handle and implement CNN-based model inference. The method proposed in the present patent application opts for the simplicity and determinism of traditional signal processing approaches as discussed below, therefore not relying on CNNs except to optionally perform a rudimentary region of interest detection or optionally a face detection.

Traditional signal processing approaches may further be divided between "blind" approaches, such as those in Poh, Ming-Zher, Daniel J. McDuff, and Rosalind W. Picard, "Advancements in noncontact, multiparameter physiological measurements using a webcam." IEEE transactions on biomedical engineering 58.1 (2010): 7-11, the entire contents of which are herein incorporated by reference, and approaches aware of the signal properties they are searching for, such as in De Haan, Gerard, and Vincent Jeanne. "Robust pulse rate from chrominance-based rPPG." IEEE transactions on biomedical engineering 60.10 (2013): 2878-2886, the entire contents of which are also herein incorporated by reference. The "blind" approaches target specifically off-line or correlation-matrix-based analysis of the input signal and, in this way, are less convenient for implementation on embedded devices.

The method according to the present invention focuses therefore on approaches aware of the properties of the signal being searched for, which are more in-line with the proposed method. As introduced previously by De Haan, Gerard, and Vincent Jeanne. "Robust pulse rate from chrominance-based rPPG." IEEE transactions on biomedical engineering 60.10 (2013): 2878-2886, the method according to the present invention also projects the input multi-dimensional signal containing colour information, such as a three-dimension RGB signal, to a two-dimensional plane.

However, instead of obtaining only a single input channel, the method according to the present invention extends the prior art by creating multiple input channels, by applying a rotation to the projected vectors (in the two-dimensional signal space obtained as described in the preceding paragraph) to generate multiple two-dimensional signal spaces.

Even though the theoretical background of remote photoplethysmography is well understood and explored in laboratories, achieving a robust solution to read heart rate from human skin, on mobile and embedded devices in real-time conditions, is a challenging task. Environmental noise, primarily comprising changes in lighting and head movement, generates major disturbances in the spatial domain and in the frequency band of interest. In addition, practical applications on embedded devices require responsiveness and short reading latency, imposing constraints on available computational performance, energy consumption, and the use of memory and the input data sizes.

The above-mentioned constraints exclude the use of "off-line" approaches such as principal component analysis or singular spectral decomposition due to their latency and computational complexity for real-time solutions expecting or requiring fine spectral resolution or large acquisition time to cope with spectral leakage and in-band noise suffer from reduced reactiveness and increased power consumption. Furthermore, solutions relying on skin-segmentation and sophisticated detection of face features may neither be applicable to embedded devices.

A goal of the present invention is to propose a method that can decrease the impact of noise and allow for robust implementation on embedded devices, for example a method of determining a heart rate of a human subject or human heart-rate determination method which operates in real-time and may be deployed in various environments, including those with lighting and movement noise, and in various hardware settings, from a smartphone to a personal computer to an embedded device or embedded platforms with limited computational resources.

A further aim of the present invention is to provide a method of determining heart rate of a human subject which is completely non-intrusive and non-contact, i.e., no hardware or sensors come into physical contact with any part of the human subject, and no involvement is required by the human subject, allowing for a heart rate reading which is completely uninfluenced by the method of determining the heart rate.

A further aim of the present invention is providing of a method of determining heart rate which may be used in a real-time telemedicine setting, for example, by implementing the method on a personal computer used by a patient during a telemedicine consultation with a doctor.

A further aim of the present invention is providing a method of determining heart rate which may be used in a non-invasive fashion, for example to determine the heart rate of a baby in a neonatal unit in a hospital, without the heart rate reading being influenced by the method of acquisition of the heart rate reading, as could be the case with contact methods.

## SUMMARY OF THE INVENTION

The present invention addresses the above-mentioned limitations of existing methods of determining heart rate using remote photoplethysmography rPPG by providing a method of determining a heart rate of a human subject according to claim 1, and a human heart-rate determination system according to claim 11.

Other advantageous features may be found in the dependent claims.

The method according to the present invention addresses the above-mentioned limitations of the prior art and shows capacity to achieve satisfactory reading accuracy and latency, and can use wide variety of video sensors, starting from VGA range of resolutions, for example, 640x480 pixels, and as low as 10 frames-per-second capture rates.

According to one aspect of the invention, a method of determining a heart rate HR of a human subject is provided. The method comprises illuminating at least one region of interest of the human subject with a light source, the light source being at a distance from the human subject; receiving airborne light reflected from the at least one region of interest; generating an input colour video signal from the received light, and, processing the input colour video signal and estimating the heart rate HR from the input colour video signal by (i) generating at least three intermediate signals IS from a portion of the input colour video signal generated from the received airborne light reflected from the at least one region of interest; (ii) projecting the at least three intermediate signals IS onto a two-dimensional skin-colour plane to create two skin-colour plane signals SCS; (iii) generating a plurality of remote photoplethysmography signals from the two skin-colour plane signals SCS; (iv) estimating a heart rate value $H_{\beta_i}$ for each remote photoplethysmography signal $rPPG_i$ based on a frequency content of each remote photoplethysmography signal, and, (v) determining the heart rate HR of the human subject based on the estimated heart rate values Hbr for each remote photoplethysmography signal $rPPG_i$; or based on the estimated heart rate values Hbr for each remote photoplethysmography signal $rPPG_i$ and a previously determined heart rate pHR, determined using the preceding method steps.

By generating a plurality of remote PPG signals, the method according to the present invention advantageously creates redundancy and reduces or eliminates in-band noise for at least some of the reading channels of the plurality of remote PPG signals.

According to a preferred embodiment of the invention, each intermediate signal of the at least three intermediate signals may comprise an average of the pixel values of each of the colour channels of the input colour video signal for the duration of the video signal.

According to a preferred embodiment of the invention, the method further comprises generating each remote photoplethysmography signal $rPPG_i$ by rotating the two skin-colour plane signals SCS by a rotation angle $\beta_i$, and combining the two rotated skin-colour plane signals according to a combination rule, wherein each generated remote photoplethysmography $rPPG_i$ corresponds to a rotation by the rotation angle $\beta_i$.

According to a preferred embodiment of the invention, the method further comprises projecting the at least three intermediate signals IS onto a two-dimensional skin-colour plane by multiplying the at least three intermediate signals IS by a projection matrix P.

According to a preferred embodiment of the invention, the method further comprises rotating the two skin-colour plane signals by the rotation angle $\beta_i$ by multiplying the two skin-colour plane signals SCS by a rotation matrix $R_{\beta_i}$ defined by the rotation angle $\beta_i$.

According to a preferred embodiment of the invention, the plurality of rotation angles may comprise a range from 0° and 180°, and each generated remote photoplethysmography signal $rPPG_i$ corresponds to a rotation by one of the rotation angles $\beta_i$ in the range.

The signal rotation step or reading channel rotation step may be used to introduce multiple reading channels of remote PPG signals, thereby bringing about a further advantage of increasing the amplitude of the frequency component corresponding to the heart beat of the human subject in the rPPG signal or signals, relative to the frequency components of the signal coming from other sources such as environmental noise, thus distinguishing the heart-beat frequency component even in the presence of in-band environmental noise.

According to a preferred embodiment of the invention, the combination rule may comprise a linear combination of the two skin-colour plane signals.

According to a preferred embodiment of the invention, the light source may comprise ambient lighting.

According to a preferred embodiment of the invention, the region of interest may comprise a face of the human subject.

According to a preferred embodiment of the invention, generating the at least three intermediate signals from a portion of the input colour video signal generated from the received light reflected from the at least one region of interest may further comprise detecting a face of the human subject in a portion of the input colour video signal.

According to a preferred embodiment of the invention, estimating a heart rate value for each remote photoplethysmography signal may comprise finding a signal frequency component with the maximum value in a frequency range of physiological interest for detecting heart rate for each remote photoplethysmography signal, and converting the frequency component in units of Hz to a heart rate value in units of beats-per-minute.

According to a preferred embodiment, the method further distinguishes itself with respect to existing solutions by introducing a penalty-and-award based heart rate detection or determination, and heart rate tracking system.

According to a preferred embodiment of the invention, the method may further comprise determining the heart rate HR of the human subject based on the estimated heart rate values Hbr for each remote photoplethysmography signal $rPPG_i$ by (i) grouping the estimated heart rate values Hbr for each remote photoplethysmography signal $rPPG_i$ into at least two clusters; (ii) characterizing each cluster in terms of an average value and a count value, wherein the average value is calculated as the average of all the estimated heart rate values belonging to the cluster, and wherein the count value is found by counting the number of estimated heart rate values belonging to the cluster; (iii) for each cluster, penalizing or reducing its count value if its average heart rate value falls within a user-defined limit HeartRateInterval starting at the lower edge LowHbr, or ending at the upper edge HighHbr, of a range of heart rate values of physiological interest; (iv) calculating the heart rate HR as the average value of the cluster with the greatest count after penalizing the counts.

According to yet another preferred embodiment of the invention, the method may further comprise determining the heart rate HR of the human subject based on the estimated heart rate values Hbr for each signal in the plurality of remote photoplethysmography signals and a previously estimated heart rate pHR by (i) grouping the estimated heart rate values Hbr for each remote photoplethysmography signal into at least two clusters; (ii) characterizing each cluster in terms of an average value and a count value, wherein the average value is calculated as the average of all the estimated heart rate values belonging to the cluster, and wherein the count value is found by counting the number of estimated heart rate values belonging to the cluster; (iii) for each cluster, either, penalizing or reducing its count value if its average heart rate value falls within a user-defined limit HeartRateInterval starting at the lower edge LowHbr, or ending at the upper edge HighHbr, of a range of heart rate values of physiological interest, or, awarding or increasing its count value if its average value is less than a user-defined absolute difference HeartRateInterval/2 from the previously estimated heart rate pHR; (iv) selecting an intermediate heart rate estimate NextHbrPick as the average value of the cluster with the greatest count after penalizing and awarding the counts; (v) calculating the heart rate HR by applying an exponential smoothing filter to the intermediate heart rate estimate NextHbrPick and a previously estimated heart rate pHR.

The usage of penalties and awards in heart rate detection brings about a further advantage wherein the heart rate determination method according to the present invention further resists environmental noise and reduces sensitivity to outliers.

According to yet another embodiment of the invention, a human heart-rate determination system is provided, comprising a camera unit configured to receive airborne light reflected from at least one region of interest of a human subject and configured to generate an input colour video signal from the received airborne light, the camera unit being at a distance from the human subject; at least one processing unit, comprising processing circuitry, individually and/or collectively configured to process the input colour video signal and estimate a heart rate HR of the human subject by the use of remote photoplethysmography, wherein the processing unit is configured to (i) generate at least three intermediate signals IS from a portion of the input colour video signal comprising the region of interest; (ii) project the at least three intermediate signals IS onto a two-dimensional skin-colour plane to create two skin-colour plane signals SCS; (iii) generating a plurality of remote photoplethysmography signals from the two skin-colour plane signals SCS; (iv) estimate a heart rate value $H_{\beta_i}$ for each remote photoplethysmography signal rPPG$_i$ in the plurality of remote photoplethysmography signals based on a frequency content of each remote photoplethysmography signal, and, (v) determine the heart rate HR of the human subject 1 based on the estimated heart rate values Hbr for each remote photoplethysmography signal rPPG$_i$; or based on the estimated heart rate values Hbr for each remote photoplethysmography signal rPPG$_i$ and a previously estimated heart rate pHR according to the preceding steps.

According to another embodiment of the invention, a computer-implemented method for performing the processing steps according to claim 1 is provided.

The above and other objects, features, and advantages of the present invention and the manner of realizing them will become more apparent, and the invention itself will best be understood from a study of the following detailed description of preferred embodiments of the invention, with reference to the attached drawings.

## BRIEF DESCRIPTION OF THE FIGURES

Figure 1 demonstrates principles of remote photoplethysmography signal acquisition and processing, as known from the prior art.

Figure 2 shows a preferred exemplary embodiment of a human heart-rate determination system using remote photoplethysmography according to the present invention.

Figure 3 shows an exemplary embodiment of the method according to the present invention comprising processing steps for processing an input colour video signal and estimating the heart rate from the processed input colour video signal (input frame).

Figure 4A is an example of at least three intermediate signals or generated from a portion of an input colour video signal according to the invention.

Figure 4B shows an exemplary remote photoplethysmography signal comprising a linear combination of the three signals plotted in Fig. 4A.

Figure 5A contains a graph that demonstrates a few seconds of the remote photoplethysography signal, for example the remote photoplethysmography signal of Fig. 4B.

Fig. 5B contains a graph that demonstrates a few seconds of a remote photoplethysmography signal obtained by projecting three intermediate signals onto a two-dimensional skin-colour plane to create two skin-colour plane signals, and combining the two skin-colour plane signals according to a combination rule, wherein the two skin-colour plane signals are rotated by an angle $\beta_i$ = 55° before being combined.

Figure 6A contains a graph that demonstrates the frequency content of the remote photoplethysmography signal plotted in Fig. 4B.

Figure 6B contains a graph that demonstrates the frequency content of the signal plotted in Fig. 5B.

Figure 7 contains an example of windowing the signals of Fig. 4A and Fig. 4B for frequency analysis which accounts for a non-stationarity of the time domain signals of Figs. 4A and 4B.

Figure 8 is a picture representing an exemplary embodiment of the human heart-rate determination system of according to an example of the present invention, with an embedded prototyping board (Synaptics Astra Board) configured to run an exemplary embodiment of the processing steps of the method according to the present invention.

Figure 9 is a flow chart of the processing steps by which a processing unit is configured to process an input colour video signal and estimate a heart rate HR of a human subject by use of remote photoplethysmography, in accordance with an embodiment of the invention.

Figure 10 is a flow chart of a method for determining a heart rate HR of a human subject, in accordance with an embodiment of the invention.

## DETAILED DESCRIPTION OF PREFERRED EMBODIMENTS OF THE INVENTION

While the invention is disclosed below with reference to certain preferred embodiments, numerous modifications, alterations, and changes to the described embodiments, and equivalents thereof, are possible without departing from the sphere and scope of the invention.

Accordingly, it is intended that the invention be given the broadest reasonable interpretation in accordance with the language of the appended claims and not be limited to the described embodiments. The features of any one of the below-described embodiments may be included in any other embodiment described herein.

The present invention concerns a human heart-rate determination system 10, shown according to an exemplary embodiment in Fig. 2. The system 10 comprises a camera unit 6, and at least one processing unit 7 comprising processing circuitry (not illustrated in Fig. 2) and configured to estimate a heart rate HR of a human subject 1 by the use of remote photoplethysmography. The human subject 1 may be, for example, an adult or elderly person of any sex and varying skin tones.

The camera unit 6 is configured to receive airborne light 4 reflected from at least one region of interest 2 of the human subject 1, the camera unit 6 being at a distance d from the human subject 1.

The at least one region of interest 2 may comprise, for example, a face or a part of the face as shown in Fig. 2, or another body part of the human subject 1, in any case comprising a blood-perfused tissue portion (not illustrated in Fig. 2), wherein small changes in colour and/or intensity of light reflected from the blood-perfused tissue portion of the at least one region of interest 2 contain information related to blood volume changes or fluctuations in a microvascular bed of tissue of the blood-perfused tissue portion from which vital signs such as heart rate may be derived.

The at least one region of interest 2 may be illuminated by light waves LW emanating from a light source 3, the light source 3 set at a distance from the human subject 1 and comprising ambient lighting (not represented in Fig. 2), wherein ambient lighting comprises sunlight and/or a light source or an artificial light source, wherein an artificial light source comprises a light source attached to a structure in a location where the human subject 1 is present, for example a light bulb in a ceiling lamp.

As the invention comprises a human heart-rate determination system by the use of remote, i.e., non-contact, photoplethysmography, the light source 3 preferably does not contact a skin (not illustrated in Fig. 2) of the human subject 1. The light or light waves LW produced by the light source 3 preferably undergo free-space propagation before the light waves LW come into contact with the skin of the human subject 1 at least the region of interest 2.

The light source 3 may preferably be set at a distance and orientation from the human subject 1 allowing the region of interest 2 to be at least partially illuminated. For example, according to an embodiment of the invention, a light source 3 comprises a ceiling lamp set at 1.5 m from the human subject 1, the region of interest 2 facing toward the ceiling lamp and also towards the camera unit 6.

The camera unit 6 is further configured to generate an input colour video signal 5 from the received airborne light 4, the camera unit 6 being at a distance d from the human subject 1, for example a distance d ranging from 20 cm to 1.5 m.

The camera unit 6 or camera may comprise for example, at least one digital video camera that continuously or at regular intervals converts light into discrete signals, capturing sequences of digital photographs of at least the at least one region of interest 2 of the human subject 1, or of an environment comprising the human subject, in digital memory.

The camera unit 6 may comprise, for example, an image sensor for capturing photographs and may further comprise for example digital memory for storing sequences of digital photographs in digital memory, for example for storing the input colour video signal 5.

The camera unit 6 may comprise, for example, an image sensor such as a charge-coupled device (CCD) or complementary metal-oxide (CMOS) digital image sensor.

The camera unit 6 may have a resolution starting at the VGA range of resolutions, for example starting at 640x480 pixels with increasing resolution requiring more memory and processing time. Therefore, the resolution should preferably be chosen as a trade-off between latency and accuracy, with low start-up latency (< 3 s) and high accuracy (95%) achievable according to the system or method described in embodiments of the present disclosure with, for example, a camera resolution of 640x480 pixels.

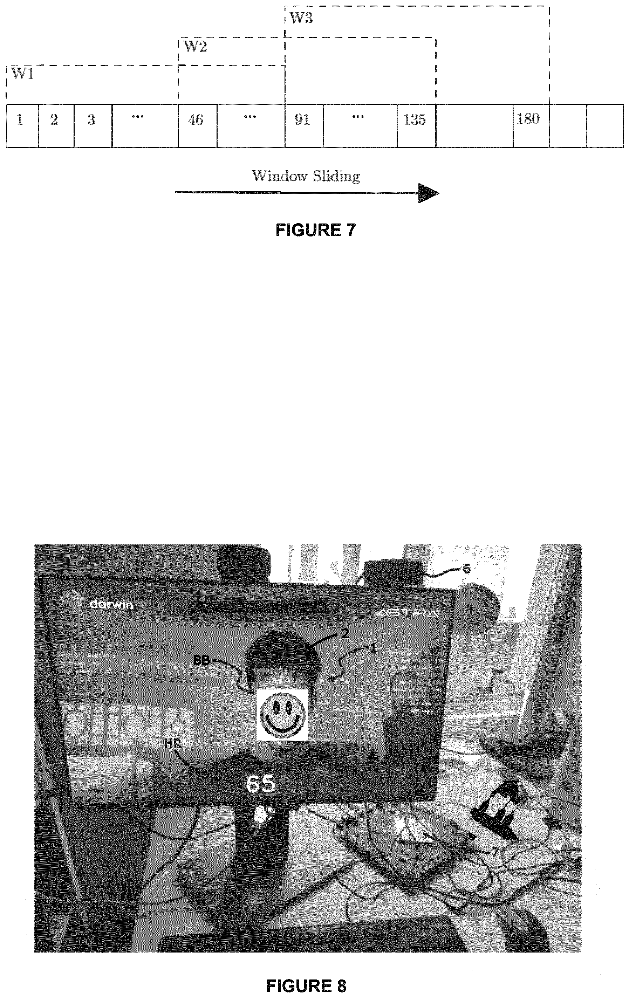

The camera unit 6 or camera may comprise, for example, a digital camera incorporated or integrated into a mobile device such as a smartphone or a purpose-built heart rate detection mobile device; a digital camera incorporated or integrated into a personal computer; or a digital camera add-on for example as shown in Fig. 8 as an add-on device to place at the top of a computer screen and configured to capture images of at least a region of interest 2 of the human subject 1.

Suitable frame sampling frequencies or frames-per-second or frame rates or capture rates may be in a range starting at 10-frames-per-second and up to 30 frames-per-second or higher. Higher frame rates require the use of more digital memory to store the input colour video signal 5 and subsequently require more processing time to be processed by a given processing unit 7.

The camera unit 6 or camera may use any image format that conveys colour information within the digitized image, for example, red-green-blue (RGB), cyan-magenta-yellow-key (CMYK), or hue-saturation-value (HSV), to generate an input colour video signal 5.

The camera unit 6 or camera may be connected to the at least one processing unit 7, for example may comprise a wireless or wired connection, for example configured to communicate the input colour video signal from the camera unit 6 to the at least one processing unit 7.

As shown in Fig. 2, the processing unit 7 is configured to receive the input colour video signal 5 generated by the camera unit 6. The processing unit 7 and the camera unit 6 may comprise a wired connection or wireless connection, for example, a USB, Ethernet, or Wifi connection.

The input colour video signal 5 comprises information including but not limited to at least one region of interest 2 of the human subject 1. The at least one region of interest 2 corresponding to a blood-perfused skin tissue portion of the human subject comprises sufficient information to estimate heart rate according to the present disclosure.

Each processor or processing unit 7 herein includes processing circuitry, and/or may include multiple processors. For example, as used herein, including the claims, the term "processor" or "processing unit" may include various processing circuitry, including at least one processor, wherein one or more of the at least one processor, individually and/or collectively in a distributed manner, may be configured to perform various functions described herein.

As used herein, when "a processing unit", "at least one processing unit", and "one or more processing units" are described as being configured to perform numerous functions, these terms cover situations, for example and without limitation, in which one processing unit performs some of recited functions and another processing unit(s) performs other of recited functions, and also situations in which a single processing unit may perform all recited functions. Additionally, the at least one processor unit may include a combination of processors or processing units performing various of the recited /disclosed functions, e.g., in a distributed manner. A processing unit may execute program instructions to achieve or perform various functions.

The at least one processing unit 7 is further configured to process the input colour video signal 5 and estimate the heart rate HR of the human subject 1 by the use of remote photoplethysmography.

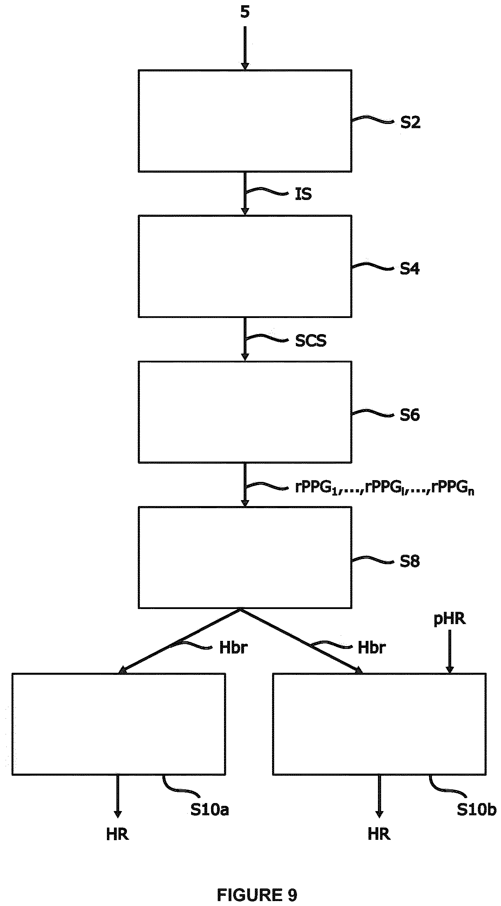

The processing steps by which the at least one processing unit 7 is configured to process the input colour video signal 5 and estimate the heart rate HR of the human subject 1 are outlined in Fig. 9 and described below.

The at least one processing unit 7 is configured in a first step S2 to generate at least three intermediate signals IS from a portion of the input colour video signal 5 comprising the region of interest 2.

The at least one processing unit 7 is further configured in a second step S4 to project the at least three intermediate signals IS onto a two-dimensional skin colour plane to create two skin-colour plane signals SCS.

The at least one processing unit 7 is further configured in a third step S6 to generate a plurality of remote photoplethysmography signals $(rPPG_1,...,rPPG_i,...,rPPG_n)$ from the two skin-colour plane signals SCS.

In a further step S8, the at least one processing unit 7 is further configured to estimate a heart rate value $H_{\beta r}$ for each remote photoplethysmography signal $rPPG_i$ in the plurality of remote photoplethysmography signal, based on a frequency content of each remote photoplethysmography signal.

The at least one processing unit 7 is further configured to determine the heart rate HR of the human subject either in a step S10a, based on the estimated heart rate values Hbr for each remote photoplethysmography signal $rPPG_i$; or in a step S10b, based on the estimated heart rate values Hbr for each remote photoplethysmography signal $rPPG_i$ and a previously estimated heart rate pHR.

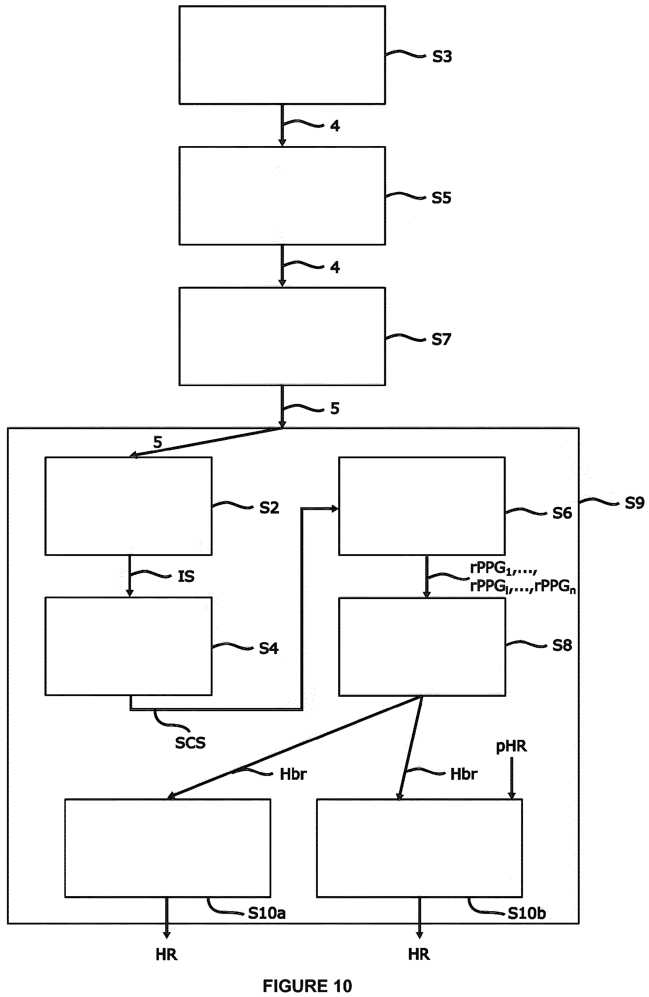

The present disclosure also concerns a method of determining a heart rate HR of a human subject 1, outlined in Fig. 10 and described below.

The method comprises in a step S3 illuminating at least one region of interest 2 of the human subject 1 with a light source 3 as shown in Fig. 2, the light source being at a distance from the human subject 1. As previously discussed, the light source 3 may comprise ambient lighting.

The method further comprises in a step S5 receiving airborne light 4 reflected from the at least one region of interest 2 as shown in Fig. 2, and in a step S7 generating an input colour video signal 5 from the received light 4. According to an embodiment of the invention, a camera unit 6 or camera is configured to receive airborne light 4 from the at least one region of interest 2 and generate an input colour signal 5 from the received light, as shown in Fig. 2 or Fig. 8 for example. The input colour video signal 5 may have a resolution starting at the VGA range of resolutions, for example starting at 640x480 pixels.

The method further comprises in a step S9 processing the input colour video signal 5 and estimating the heart rate HR from the input colour video signal 5, wherein step S9 comprises performing the steps S2, S4, S8, and either S10a or S10b. According to an embodiment of the invention, at least one processor or processing unit 7 comprising processing circuitry is configured to perform step S9 (and therefore configured to perform the steps S2, S4, S8, and either S10a or S10b, that S9 comprises), as shown in Fig. 2 or Fig. 8.

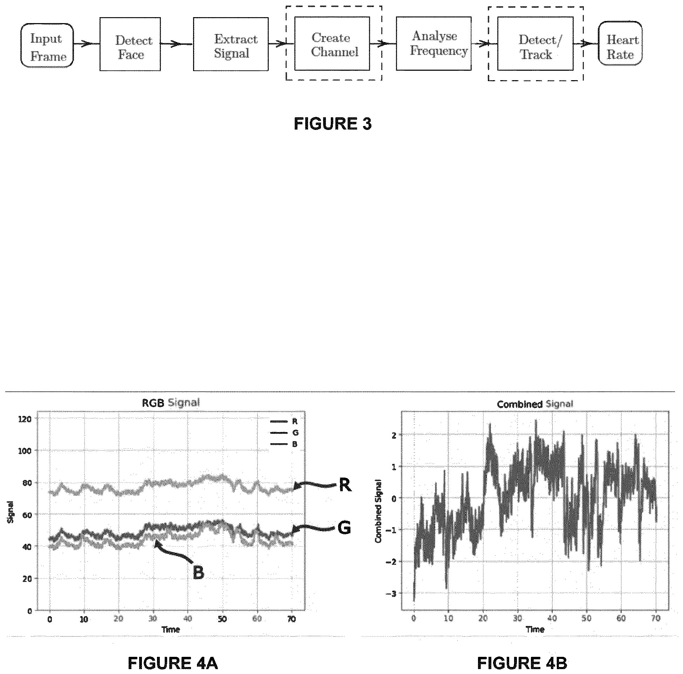

The method according to an exemplary, non-limiting embodiment of the invention demonstrated in Fig. 3 may comprise, for example, the following blocks:

- Simple face detection providing bounding box outputs; no need for face landmarks
- Signal extraction providing average value of input video channels
- Signal projection and channels creation combining the face colour components into a single input signal
- Frequency analysis for all channels
- Head pose and lightness analysis
- Penalty-and-award-based heart rate detection and tracking

The detailed implementation of method step S9 is discussed below.

The method step S9 comprises an initial step S2, wherein S2 comprises generating at least three intermediate signals IS from a portion of the input colour video signal 5 generated from the received airborne light 4 reflected from the at least one region of interest 2.

According to an embodiment of the invention wherein the region of interest 2 comprises a face of the human subject, the step S2 may optionally comprise detecting a portion of the input colour video signal 5 comprising the face of the human subject 1, or detecting for example a bounding box comprising the face of the human subject, wherein a bounding box, the location of which in an image is defined by pixel coordinates, comprises a portion of the input colour video signal substantially comprising the face, see for example BB in Fig. 8.

Face and landmark detection AI models, (see, for example, Deng, Jiankang, et al. "Retinaface: Single-shot multi-level face localisation in the wild." Proceedings of the IEEE/CVF conference on computer vision and pattern recognition. 2020) are important input blocks of many computer vision applications, such as face recognition, face classification, pain and emotion detection, people counting. For example, state-of-the-art models like RetinaFace and TinaFace are capable of accurate detection of hundreds of faces from a single input image.

However, in an application requiring detection of a region of interest 2 comprising a face of a human subject, optimized models (see, for example, the model described in Milosevic, Uros, et al. "Improving Efficiency of RetinaFace Models by Controlled Branch Pruning." 2023 31st Telecommunications Forum (TELFOR). IEEE, 2023, the entire contents of which are herein incorporated by reference), which trade-off accuracy and the number of faces which can be detected for faster execution speed and smaller model size, may be more suitable, especially for real-time applications having low latency requirements and/or applications having hardware constraints.

Therefore, according to an exemplary, non-limiting embodiment of the invention, an optimized face detection model may optionally be used to detect a face or detect a face bounding box or bounding box of the face. A relatively small input tile size, or size, for the bounding box or detected face region may be used, such as 320x240 pixels or less, favouring execution speed and efficiency.

As an additional entirely optional step, the optimized face detection models may additionally be quantised to integer formats using a post-training process similar to the procedures described in Lee, Jemin, et al. "Quantune: Post-training quantization of convolutional neural networks using extreme gradient boosting for fast deployment." Future Generation Computer Systems 132 (2022): 124-135, the entire contents of which are herein incorporated by reference, in order to facilitate their execution on neural-network hardware accelerators. Chip manufacturers, for example NXP and Synaptics, provide such hardware support regularly on their flagship embedded products, (see, for example, NXP. i.MX application processors. https://www.nxp.com/products/processors andmicrocontrollers/arm-processors/i-mx-applications-processors).

The bounding box obtained from the optional face detection or bounding box detection process may determine a human face location in the input image coordinates. According to an exemplary embodiment of the invention, the box and the input image may be used to define a subset of pixels within the bounding box for the signal extraction process. The subset may be defined, for example, using offsets relative to the bounding box boundaries. In this way, this exemplary embodiment of the invention provides a further advantage of compressing the area of interest and reducing the impact of including face-unrelated noise from the background into the signal of interest.

The optional face detection, or bounding box detection, wherein the bounding box comprises a portion of the input colour video signal, defined by pixel coordinates substantially comprising the face, brings about a further advantage of reducing memory requirements and speeding up processing time by generating the at least three intermediate signals IS from a reduced size video signal (for example 320x240 pixels vs 640x480 pixels) comprising a digitized representation of a only a blood-perfused tissue portion, and eliminating unnecessary portions from the input colour video signal 5 that could comprise further environmental noise.

According to an embodiment of the invention, a portion of the input colour video signal 5 generated from the received airborne light 4 reflected from the input colour video signal may comprise, for example, a detected face bounding box or bounding box of the face as just described.

The processing of the input colour video signal 5 comprises generating at least three intermediate signals IS from a portion of the input colour video signal 5 generated from the received airborne light 4 reflected from the at least one region of interest 2, or extracting at least three intermediate signals IS from a portion of the input colour video signal 5 generated from the received airborne light 4 reflected from the at least one region of interest 2, the implementation of which is described below.

According to an embodiment of the invention, each intermediate signal of the at least three intermediate signals comprises an average of the pixel values of each of the colour channels of the input colour video signal for the duration of the video signal. This advantageously performs a data compression step while retaining information related to blood volume changes in a blood-perfused tissue portion of the at least one region of interest, and so contributes to further reducing latency while maintaining high heart-rate estimation performance.

The at least three intermediate signals IS may be generated from the input colour video signal 5 or from a portion of the input colour video signals 5. For example, according to an embodiment of the invention, the at least three intermediate signals may be generated from a portion of the input colour video signal 5 comprising a detected face bounding box, e.g., the bounding box pixel coordinates may be provided to delimit a reduced size video signal from which to generate the at least three intermediate signals IS instead of the entire input colour video signal 5. An example of such a bounding box is demonstrated in Fig. 8.

By generating or extracting the at least three intermediate signals, the method according to the present invention generates time-based series obtained by averaging the input image planes for the region of interest or averaging the input image planes for a portion of the input colour video signal 5, for example comprising a detected face bounding box. By input image plane, it is meant the planes comprising colour information.

For example, if the RGB colour model is used to convey colour information in the input colour video signal 5, or a portion of the input colour video signal 5, the input colour video signal 5 comprises three colour planes for each frame or captured image making up the duration of the input colour video signal sequence: one red plane with pixel values representing a red contribution, one green plane with pixel values representing a green contribution, and one blue plane with pixel values representing a blue contribution to the colour image or frame.

While RGB is one example, the intermediate signal IS generation or extraction could be performed for any image format that conveys colour information within the input colour video signal 5, for example the CMYK, RGBA, or CMY colour model. The at least three intermediate signals IS generated from the input colour video signal 5 or the at least three time series extracted from the input colour video signal 5 contain information related to blood volume changes and as such are a good source or starting point for estimating the heart rate non-invasively or without contacting the human subject 1.

Following step S2 comprising generating at least three intermediate signals IS, the processing step S9 comprises step S4, comprising projecting the at least three intermediate signals IS onto a two-dimensional skin-colour plane to create two skin-colour plane signals SCS.

The input IS provided by signal extraction or at least three intermediate signal generation step S2 comprises a time series of at least three-element vectors, for example in the case of the RGB colour model, $v_i = [r_i g_i b_i]^T$. Therefore, for a time series, a sequence of vectors $v_i$ comprises a data window matrix $D$ of size $3 \times n$, where $n$ is the size of the window expressed as the number of samples, for the RGB colour model. For a colour model comprising four colour coordinates for example, the data window matrix $D$ would be of size $4 \times n$. The non-limiting RGB colour model, or a model comprising three colour coordinates, will be assumed for the remainder of the explanation.

In the projection step S4, a projection matrix P, as proposed for example in De Haan, Gerard, and Vincent Jeanne. "Robust pulse rate from chrominance-based rPPG." IEEE transactions on biomedical engineering 60.10 (2013): 2878-2886, may be used that maps the at least three intermediate signals IS comprising for example a three-dimensional input to a skin-color plane represented by $S$ of size $2 \times n$:

$$S = P \times D$$

or in more detail:

$$\begin{bmatrix} y_0 & y_1 & \cdots & y_{n-1} \\ z_0 & z_1 & \cdots & z_{n-1} \end{bmatrix} = \begin{bmatrix} p_{r0} & p_{g0} & p_{b0} \\ p_{r1} & p_{g1} & p_{b1} \end{bmatrix} \times \begin{bmatrix} r_0 & r_1 & \cdots & r_{n-1} \\ g_0 & g_1 & \cdots & g_{n-1} \\ b_0 & b_1 & \cdots & b_{n-1} \end{bmatrix}$$

where $r_i$, $g_i$, $b_i$ comprise mean values of the red, green, and blue channels as explained previously obtained in step S2, respectively; and the elements of the $P$ matrix, $p_r$, $p_g$, $p_b$, comprise projection coefficients for each of the channels; and $y, z$ are the projected signals in the two-dimensional plane, or the two skin-colour plane signals SCS. An example of suitable projection coefficients may be as follows: [0 1 -1; -2 1 1], as suggested by Wenjin Wang, et. al. "Algorithmic principles of remote PPG." IEEE Transactions on Biomedical Engineering, 64(7):1479-1491, 2017, however these values are given as an example only and the projection coefficients may be configured by the user, for example to emphasise different colour channels, for example the green channel in the RGB model.

Therefore, projecting the at least three intermediate signals onto a two-dimensional skin-colour plane comprises multiplying the at least three intermediate signals by a projection matrix to project the at least three intermediate signals, or apply a linear transformation to the at least three intermediate signals to obtain two signals $y$, $z$, referred to as skin-colour plane signals SCS.

This step S4 brings about a further advantage of further compressing the information of the input colour video signal 5 while still keeping enough relevant information to detect modulations of blood volume in a blood-perfused tissue portion that are related to heart rate.

In order to motivate the next step S6, it may be considered that the output of the projection SCS may be used to blend the two signals $y$ and $z$ and form a single output time-series or single output remote photoplethysmography signal, and then feed the single signal it to a frequency analysis part. Figure 4A shows an example of the extracted RGB signal over time according to an exemplary embodiment of the invention, together with a corresponding single output remote photoplethysmography signal in Fig. 4B-a linear combination, for example, of the $y$ and $z$ components, or the two skin-colour plane signals SCS, of the projection output, for example, the three intermediate signals of Fig. 4A multiplied by a projection matrix $P$, may create a combined signal or remote photoplethysmography signal $x$ and provide input for heart rate detection:

$$x = \alpha \cdot y + \gamma \cdot z$$

with $\gamma = 1 - \alpha$. The combination coefficients in the preceding equation may be, for example user-configurable ($\alpha$) or may depend on a ratio of standard deviations of $y$ and $z$ ($\gamma$), see, for example, Wenjin Wang, Albertus C. den Brinker, Sander Stuijk, and Gerard de Haan. Algorithmic principles of remote PPG. IEEE Transactions on Biomedical Engineering, 64(7):1479-1491, 2017. Example values for $\alpha$ and $\gamma$ are 0.5, though as mentioned, these values are user configurable. The method may comprise variance estimation, normalization and standardization, as mentioned in the just cited article, supporting the choice of $\alpha$ and $\gamma$ factors.

However, having only a single channel or a single remote photoplethysmography signal as just demonstrated for frequency estimation may hinder the development of a robust detection algorithm, making it prone to noise sources of different kind. In addition, the small number $n$ of the input data that is usually available or that practitioners tend to achieve-having in mind the method reactiveness, latency, and applicability to embedded devices-reduces frequency resolution and exposes the detection algorithm to the presence of out-of-band noise.

Therefore, to cope with the above-mentioned problems of using only a single channel or single remote photoplethysmography signal, the method according to the present invention advantageously comprises in the processing step S9 a step S6 comprising generating a plurality of reading channels of remote photoplethysmography signal, or generating a plurality of remote photoplethysmography signals from the two skin-colour plane signals SCS, instead of using only a single PPG signal for heart rate determination as in prior art, when processing the input colour video signal and estimating the heart rate from the input colour video signal.

According to an embodiment of the invention, this step S6 advantageously confers to the human heart-rate estimation method according to the present invention increased robustness to environmental noise, compared to using only a single remote photoplethysmography reading channel.

According to an embodiment of the invention, generating each remote photoplethysmography signal rPPG$_i$ of the plurality of remote photoplethysmography signals comprises rotating the two skin-colour plane signals SCS by a rotation angle $\beta_i$ and combining the two rotated skin-colour plane signals according to a combination rule, wherein each generated remote photoplethysmography signal rPPG$_i$ corresponds to rotation by the rotation angle $\beta_i$ of the two skin-colour plane signals SCS followed by combining the rotated skin-colour plane signals SCS according to a combination rule.

The combination rule discussed in the previous paragraph may comprise a linear combination of the two rotated skin-colour plane signals, such as shown in the equation above for finding x, or may comprise a non-linear combination, of the two skin-colour plane signals.

Rotating the two skin colour plane signals SCS by the rotation angle $\beta_i$ may comprise, for example, multiplying the two skin-colour plane signals SCS by a rotation matrix $R_{\beta_i}$ defined by the rotation angle $\beta_i$. For example, the full projection equation according to an embodiment of the invention comprises the following:

$$S = R_\beta \times P \times D$$

where $R_\beta$ comprises a $2 \times 2$ rotation matrix for a given angle $\beta$, defined as follows:

$$R = \begin{bmatrix} \cos\beta & -\sin\beta \\ \sin\beta & \cos\beta \end{bmatrix}$$

where a rotation matrix comprises a transformation matrix that is used to perform a rotation in Euclidean space.

By rotating the two skin-colour plane signals obtained by the matrix multiplication $P \times D$ over, for example, a range from 0° to 180°, the method according to the present invention may create additional reading channels or a plurality of remote photoplethysmography signals for subsequent analysis such as frequency analysis.

For example, according to an embodiment of the invention, having $\beta$ in 0°, 5°, 10°, ...,175° allows for creation of 36 reading channels in total. In this exemplary embodiment, the original rotation matrix comprises:

$$R_{5°} = \begin{bmatrix} \cos 5° & -\sin 5° \\ \sin 5° & \cos 5° \end{bmatrix} = \begin{bmatrix} 0.996195 & -0.0871557 \\ 0.0871557 & 0.996195 \end{bmatrix}$$

The entire range of rotation matrices may, for example, then be obtained recursively through matrix multiplications:

$$R_{\beta_{i+1}} = R_{5°} \cdot R_{\beta_i} = R_{5°}^{i+1}$$

where according to the above exemplary embodiment, $i = 0, 1, ..., 35$, $\beta_i = \{0°, 5°, ..., 175°\}$, and $R_{0°}$ comprises the $2 \times 2$ identity matrix.

The advantages of the multiple channel creation or the generation of a plurality of remote photoplethysmography signals from the two skin-colour plane signals according to step S6, and the further advantages brought about by rotating the two skin-colour plane signals by a plurality of angles before combining them comprise creating redundancy and reducing or eliminating in-band noise for some projections and are demonstrated below.

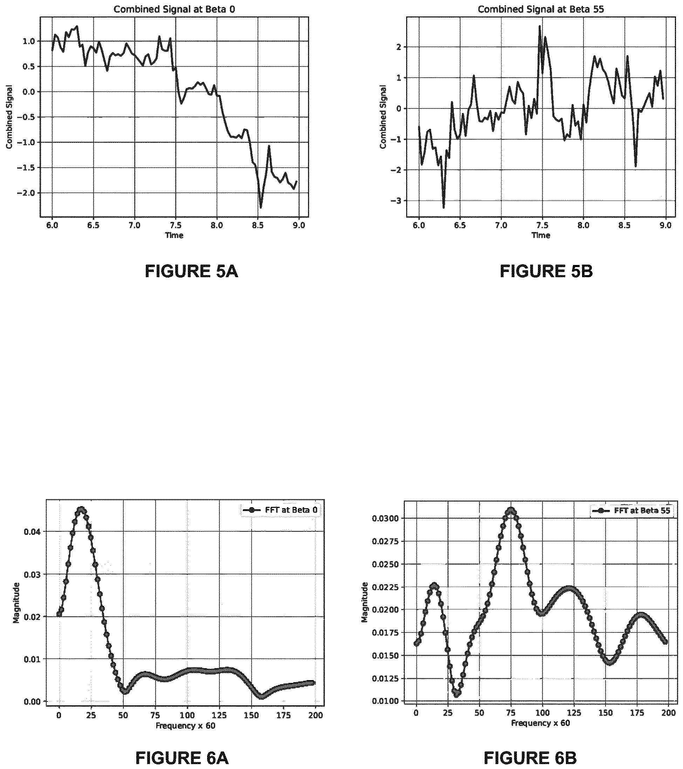

Figure 5A shows a remote photoplethsymography input signal for the original channel (at 0°), i.e., rPPG$_0$, for no rotation applied to the two-skin colour plane signals SCS before combining them, while Fig. 5B shows a remote photoplethysmography input signal obtained after applying a rotation of 55° to the two skin-colour plane signals SCS before combining them.

The filtering effect of removing the down-slope trend of unrotated signal in Fig. 5A may be observed by comparing it to the signal of Fig. 5B which was obtained by applying a rotation of 55° to the two skin colour plane signals SCS before combining them.

The particular advantage of this filtering effect for determining heart rate according to the present invention is illustrated by comparing Fig. 6A and Fig. 6B, which illustrate the signals of Fig. 5A and Fig. 5B in the frequency domain, respectively. Notably, the frequency component around 75 heartbeats per minute is hardly distinguishable in the frequency domain of the channel at 0° in Fig. 6A, while after the rotation by 55° the same spectral component at around 75 heartbeats per minute becomes dominant. It may be noted that multiplying the frequency resolution (expressed in Hz) by 60 maps it to the heartbeat range (expressed in beats-per-minute).

It is therefore easier, using simple frequency domain peak-detection analysis methods, to determine a human heart rate using the rotated and combined remote photoplethysmography signal of Fig. 5B according to an embodiment of the invention than using the signal of Fig. 5A, especially in the presence of in-band noise, wherein in-band noise here means for example electrical noise falling in the same frequency band as signals of interest for vital sign detection or heart rate detection, such as noise introduced due to fluctuations in lighting or ambient lighting, or movement of a region of interest of a human subject such as head movement.

The processing step S9 further comprises a step S8 which comprises estimating a heart rate value $H_{\beta_i}$ for each remote photoplethysmography signal rPPG$_i$ based on a frequency content of each remote photoplethysmography signal.

The frequency content of each remote photoplethysmography signal may be found using, for example, methods based on the Fourier transform, such as a discrete Fourier transform or short-time Fourier transform or modified periodogram using Welch's method.

For example, according to an embodiment of the invention, finding the frequency content of each remote photoplethysmography signal comprises applying a discrete Fourier transform to each remote photoplethymosgraphy signal rPPG$_i$, in order to find frequency components related to the blood volume changes and therefore related to heart rate.

The observed signal rPPG$_i$ is not stationary by its nature, therefore it may be advantageous to optionally use frequency analysis methods which account for the non-stationary nature of each remote photoplethysmography signal, for example, by employing signal windowing or using for example a short-time Fourier transform.

An example of short-time frequency analysis, in which the input signal may be organised in a sequence of overlapping-in-time windows, is shown in Fig. 7. Figure 7 demonstrates how a window sliding across the input signal buffer, or a sliding window, can create series of input windows for the frequency analysis. The sliding window may comprise a length, for example L=90 samples, and a window slide of, for example L/2 = 45 samples. Such a window slide, for example as shown in Fig. 7, may slide from its position at W1 including samples 1 to 90 to a second position at W2 starting at sample 46 and ending at sample 135, wherein the frequency analysis may be performed separately on windows W1 and W2.

As mentioned, in the particular example demonstrated in Fig. 7, the window size or length comprises L=90 samples and the window slides across the signal by L/2=45 samples, therefore in this example, consecutive windows overlap by 45 samples, i.e., the last 45 samples of W1 correspond to the first 45 samples of W2, for example, however this is only an example and alternative window lengths and slides are possible. In this example, for a sampling rate of 30 frames-per-second and after an initial delay of 3 seconds for acquiring the first full window of L=90 samples (corresponding to 3 seconds of signal duration), the windowing procedure as just described may provide a ninety-sample length input every 1.5 seconds for windowed frequency analysis. Longer windowed signal durations are possible, for example 7-seconds, corresponding to L=210 samples for a sampling frequency of 30 Hz.

Each remote photoplethysmography signal rPPG$_i$ may be windowed as described above, and in addition may be zero-padded, i.e., zeros may be added to the end of each remote photoplethysmography signal rPPG$_i$ for example to make the overall length of the signal equal a power of two, before finding the frequency content of the remote photoplethysmography signal rPPG$_i$, for example using a discrete Fourier transform as shown below:

$$X_\beta(k) = \sum_{n=0}^{N-1} x_\beta(n) \cdot e^{-j2\pi \cdot n \cdot k / N}$$

where in the above example, $X_\beta(k)$ comprises the spectral component for a given $k = 0, 1, ..., N - 1$ and $x_\beta(n)$ comprises a windowed (optional) and zero-padded (optional) input signal, or remote photoplethysmography signal, obtained by projecting the two skin-colour plane signals SCS with $R_\beta$, and then combining the SCS according to a combination rule.

The resulting frequency resolution in this example is determined by the $f_s/N$ ratio, where $f_s$ comprises the sampling frequency, and N comprises the number of points at which the discrete Fourier transform is computed, for example 1024 for a 1024-point DFT as is commonly used. The number of frames per second arriving from the video source defines the sampling frequency $f_s$, while $N$ may be configured by a user as part of the configuration settings, for example depending on the level of non-stationarity the user expects in each remote photoplethysmography signals, with greater N being appropriate when less signal variation is observed over time, for example.

As an example only, the windowed signal $x_\beta(n)$ may be of a duration of 3-7 seconds, or 90 to 210 samples for a sampling rate of 30 Hz, and may be zero-padded to obtain a signal with 1024 samples before computing the DFT.

It may be noted that multiplying the frequency resolution (expressed in Hz) by 60 maps it to the heartbeat range (expressed in beats-per-minute). In addition, it may be noted that the fast Fourier Transform (FFT) algorithm may be used to efficiently compute the values of all the spectral components when finding the frequency content of each remote photoplethysmography signal $rPPG_i$ using a discrete Fourier transform.

According to an embodiment of the invention, estimating a heart rate $H_{\beta_i}$ for each remote photoplethysmography signal based on a frequency content of each remote photoplethysmography signal $rPPG_i$ comprises finding, for each remote photoplethysmography signal, a signal frequency component with the maximum value in a frequency range of physiological interest for detecting heart rate, and converting the frequency component with the maximum value in units of Hz to a heart rate value in units of beats-per-minute.

A frequency range of physiological interest may comprise for example a range from 0.6 to 4 Hz and a preferred range from 0.65 to 3.0 Hz, corresponding to a range of heart rate values from 36 to 240 beats-per-minute and a preferred range from 39 to 180 beats-per-minute.

Once the frequency content of each remote photoplethysmography signal $rPPG_i$ is found using any of the suitable methods described above, out-of-band frequency components residing under low and beyond high heart rate frequencies may optionally be eliminated in each $rPPG_i$ signal by simply zeroing the out-of-band frequency components before employing max or peak-detection algorithms for detecting in-band leading frequency components, i.e., for detecting frequency components in each remote photoplethysmography signal corresponding to heart rate frequencies and from which a heart rate value $H_{\beta_i}$ may be estimated, wherein each remote photoplethysmography signal $rPPG_i$ comprises the maximum signal power at a signal frequency component with the maximum value in a physiologically interesting frequency range.

According to an embodiment of the invention, for a given plurality of remote photoplethysmography signals or given window w of a plurality of remote photoplethysmography signals, a vector of channel readings Hbr may be created, comprising an estimated heart rate value $H_{\beta_i}$, one for each of the rotation angles, for example one for each rotation angle in the range from 0° to 175°, with a step of 5° between angles.

$$Hbr(w) = \begin{bmatrix} H_{\beta_0} & H_{\beta_1} & \cdots & H_{\beta_K} \end{bmatrix}^T$$

where $H_{\beta_i}$ is the estimated heart rate value for a given remote photoplethysmography signal $rPPG_i$ corresponding to a given projection angle $\beta_i$ from the previous step S6.

The processing step S9 further comprises determining the heart rate HR of the human subject 1 either in a step S10a if a previously determined heart rate pHR is not available, e.g., in a first iteration of the method steps upon program start-up, or in a step S10b if a previously determined heart rate pHR is available, e.g. at least one iteration of the method steps has already been performed.

The method step S10a for determining the heart rate HR of the human subject when a previously determined heart rate pHR is not available is first described below. This step could be performed during a first iteration of method steps S3, S5, S7, and step S9 comprising steps S2, S4, S6, and S8. The step S10b for determining the heart rate HR of the human subject 1 when a previously determined heart rate pHR is available, i.e., if S10a has already been performed in a first iteration of the method steps, will then be described.

According to an embodiment of the invention, step S10a for determining the heart rate HR of the human subject 1 based on the vector of estimated heart rate values Hbr for each remote photoplethysmography signal $rPPG_i$ comprises a sequence of steps which may be programmed in a computer programming language, such as Python or C++, and implemented on at least one processing unit 7.

According to an embodiment of the invention, step S10a first comprises grouping the estimated heart rate values Hbr for each remote photoplethysmography signal $rPPG_i$ into at least two clusters, wherein a cluster comprises, for example, heart rate estimates that are closer in value to the average heart rate value of the cluster than to an average heart rate value of another cluster. The grouping or clustering of the values comprising the estimated heart rate values Hbr may be performed using a clustering algorithm such as the k-means algorithm or an optimized version of the k-means algorithm (see, for example, Potera Ş, Cosmin Marian, Marian Cristian Mihaescu, and Mihai Mocanu. "An optimized version of the k-means clustering algorithm." *2014 Federated Conference on Computer Science and Information Systems.* IEEE, 2014).

The step S10a then comprises characterizing each cluster in terms of an average value and a count value, wherein the average value is calculated as the average of all the estimated heart rate values belonging to each cluster, and wherein the count value is found by counting the number of estimated heart rate values belonging to each cluster.

The step S10a then comprises a penalizing or count-reduction step, comprising for each cluster, penalizing, i.e., reducing, its count value if its average heart rate value falls within a user-defined limit HeartRateInterval starting at a lower edge LowHbr or ending at an upper edge HighHbr, of a range of heart rate values of physiological interest.

This penalizing or count-reduction step has the advantage of increasing method robustness to outlier estimated heart rate values for each remote photoplethysmography signal $rPPG_i$, for example arising due to environmental noise or human subject 1 movement while acquiring the input colour video signal 5.

The step S10a finally comprises selecting the human heart rate HR as the average value of the cluster with the highest count after penalizing the counts, i.e., after the previous penalizing or count-reduction step.

Step 10b is substantially similar to step S10a and is outlined according to an embodiment of the invention below.

According to an embodiment of the invention, step S10b first may comprise grouping the estimated heart rate values Hbr for each remote photoplethysmography signal $rPPG_i$ into at least two clusters, wherein a cluster comprises, for example, heart rate estimates that are closer in value to the average heart rate value of the cluster than to an average heart rate value of another cluster.

According to an embodiment of the invention, step S10b may then comprise characterizing each cluster in terms of an average value and a count value, wherein the average value is calculated as the average of all the estimated heart rate values belonging to each cluster, and wherein the count value is found by counting the number of estimated heart rate values belonging to each cluster.

Step S10b may further comprise a penalty and award step, i.e., for each cluster, either, penalizing or reducing its count value if its average heart rate value falls within a user-defined limit HeartRateInterval starting at a lower edge LowHbr, or ending at an upper edge HighHbr of a range of heart rate values of physiological interest; or awarding or increasing the count value of the cluster if its average value is less than a user-defined absolute difference (HeartRateInterval/2) from the previously estimated heart rate pHR according to the preceding steps.

The usage of penalties and awards in heart rate detection brings about a further advantage wherein the heart rate determination method according to the present invention further resists environmental noise and reduces sensitivity to outliers by reducing the contribution of estimated heart rate values close to edges of a heart rate interval of physiological interest, and/or weighting favourably heart rate estimations that are close to previously estimated heart rates.

The parameters LowHbr, HighHbr, HeartRateInterval, EdgeHbrPenalty, and MinDiffAward may be, for example, configured by a user upon program startup. LowHbr may be for example in a range between 35 and 60 beats-per-minute, HighHbr may be, for example, in a range between 180 and 240 beats-per-minute, and HeartRateInterval may be, for example, in a range between 5 and 30 beats-per-minute. These parameters may for example be tailored for different target ages or sexes. EdgeHbrPenalty may be for example in a range between 1.5 and 3.5, while MinDiffAward may be, for example, in a range between 1.25 and 3.0. These parameters could also, for example, be tailored to different environments, for example, environments where it is known that more or less noise is present. These values are configurable and the above ranges are given only as non-limiting examples only.

The previously estimated heart rate pHR may have been calculated in an initial iteration of the method steps that step S10a comprises, or in a subsequent iteration of the method steps comprising step S10b, and the previously estimated heart rate may be available or stored in program memory.

Step S10b may further comprise selecting the heart rate HR as the average value of the estimated heart rate values belonging to the cluster with the highest count after penalizing and awarding the counts of each cluster as previously described.

Step S10b may further comprise selecting or updating the heart rate HR by applying an exponential smoothing filter to the (i) average value of the estimated heart rate values belonging to the cluster with the highest count after penalizing and awarding the counts of each cluster as previously described NextHbrPick and a (ii) previously estimated heart rate pHR.

For illustrative purposes, an exemplary, non-limiting embodiment of step S10b for detecting and tracking a human heart rate is described below and shown in pseudo-code in Algorithm 1, where the preceding method steps are assumed to have been already performed.

According to an embodiment of the invention, step S10b comprises a heart rate detection or determination and tracking algorithm, using the vector Hbr generated for all remote photoplethysmography signals $rPPG_i$ to estimate heart rate at a given time instant, and Hbr produced consecutively across all input signal windows, analyzing one window per main processing loop, to track and provide heart rate estimation in real time.

An example of procedural steps used for the heart rate detection and tracking according to an embodiment of step S10a and S10b are shown in Algorithm 1. Lines 1-6 illustrate an example of program or algorithm initialization, while lines 7-15 depict an example of the main processing loop. The initialization phase in lines 1-6 sets for example major operating conditions, such as number of channels to create or number of remote photoplethysmography signals $rPPG_i$ to generate by defining the range of rotation angles or through the angles range, and/or for example parameters for awards and penalties such as EdgeHbrPenalty and MinDiffAward. The initialization phase may also define for example the heart rate band of interest using low and high heartbeat markers, e.g., LowHbr, and HighHbr, and may also specify the heart rate search interval, e.g., HeartRateInterval.

According to an embodiment of the invention, the main processing loop may read an input-signal window and process it until interrupted or until the input-buffer is emptied.

The main processing loop may perform frequency analysis for the remote photoplethysmography signals generated for the entire range of angles, as explained in for example in steps S6 and S8. As previously explained, the return or output value of step S8 comprises an array of heart rate estimations Hbr for each of the created channels or each of the remote photoplethysmography signals rPPG$_i$ for each of the rotation angles $\beta_i$. A first-in-first-out (FIFO) data structure may preserve the channels history for example for the last L readings. Having the history trace for each of the channels may be helpful for channel quality estimation or for outliers elimination, for example.

| | **Algorithm 1** Heart Rate Detection and Tracking |
|---|---|
| **1** | *PreviouslyEstimatedHeartRate (pHR)* ← Not Available |
| **2** | *HbrChannelHistory* ← FIFO Structure of size L |
| **3** | *BetaRange* ← from $\beta_{start}$ to $\beta_{stop}$ using $\beta_{step}$ |
| **4** | *LowHbr, HighHbr, HeartRateInterval* ← ConfigurationRead |
| **5** | *EdgeHbrPenalty, MinDiffAward, $\zeta$* ← ConfigurationRead |
| **6** | *w ← ReadCircularBuffer()* |
| **7** | **while** *w* not empty **do** |
| **8** | *Hbr ← FrequencyAnalysys(w, BetaRange)* |
| **9** | *HbrChannelHistory.push(Hbr)* |
| **10** | *Hbr ← Agglomerate(Hbr, HeartRateInterval)* |
| **11** | *Hbr ← Penalties(Hbr, HeartRateInterval, LowHbr, HighHbr, EdgeHbrPenalty)* |
| **12** | **if** *pHR available* **do** |
| **13** | *Hbr ← Awards(Hbr, HeartRateInterval, pHR, MinDiffAward)* |
| **14** | *NextHbrPick ← TopSelection(Hbr)* |
| **15** | *HeartRate (HR) ← $\zeta \cdot$ NextHbrPick +(1 − $\zeta$) $\cdot$ pHR* |
| **16** | *w ← ReadNextWindow()* |

According to an embodiment of the invention, the next step in the loop performs the process of agglomeration or clustering. It may group or agglomerate or cluster the estimated heart rates detected from different channels, i.e., the heart rate estimates $H_{\beta_i}$ for each of the remote photoplethysmography signals rPPG$_i$ making up the vector Hbr, around a central heart rate value or average heart rate value, for example should their distance from one another be within a user-defined search interval HeartRateInterval. The average heart rate of the heart rate estimates of each of the clusters along with the count of the detections within the search interval or within each of the clusters characterises each of the formed clusters.

Heart rate estimates belonging to a given cluster are for example closer in value to the average heart rate value of the cluster than to an average heart rate value of another cluster. As previously discussed, the clustering may be performed using a suitable clustering algorithm, such as the k-means algorithm.

According to the embodiment of step S10b described in Algorithm 1, after identifying the candidate heart rates, i.e., the average heart rates of each of the clusters, the counts of the clusters may be used to apply "penalties" and "awards," to the counts, for example, increasing or decreasing the count value of each of the formed clusters, for example according to the following procedure.

As a motivation for the penalty and award step, it may be noted that in cases when a transitory, in-band noise is present, for example due to environmental noise or human subject movement, heart rate readings close to the band edges may appear in some channels, for example, the heart rate estimated for some of the remote photoplethysmography signals rPPG$_i$ may be close or within a user-defined difference of an upper or lower end of a range of heart rate values of physiological interest, for example ranging from 39 to 180 beats-per-minute.

Therefore, if an agglomeration or cluster comprises an average heart rate value close to the band edges (low or high), its count may be penalised, for example may be scaled down or reduced by a user-defined penalty factor EdgeHbrPenalty. In the same way, for agglomerations or clusters comprising an average heart rate value close to a previously estimated heart rate pHR, their counts may be awarded and scaled up or increased by the user-defined award factor MinDiffAward.

The penalizing and awarding procedure provides a further advantage of eliminating noise and improving the reading robustness, without losing reactiveness and convergence. Accordingly, the procedure may select or determine the human heart-rate HR that most-likely provides the best fit to the physiological state of the observed person or human subject.

As a final step of Algorithm 1, the first-order IIR filtering or exponential smoothing filter of line 14 prepares the output heart-rate HR value as an exponential average of a previously estimated heart rate pHR and the average heart rate of the cluster with the highest count after the penalty and award procedure NextHbrPick of the method according to the embodiment represented in Algorithm 1 returned back to the user, where the smoothing factor $\zeta$ may be configured by the user, for example in a non-limiting range between 0.7 and 1.0.

Sliding the input window forward for the user-defined amount of time (as depicted in Figure 7) may prepare the next input and continue the heart rate estimation or determination for subsequent windows, until the input stream of windows is over or interrupted.

## 4 Implementation

The method according to the invention, according to steps S3, S5, S7, and S9 comprising steps S2, S4, S6, S8, and either S10a or S10b may be implemented and deployed on for example several embedded and mobile platforms. By embedded platform or system or device it is meant a specialized computer system, for example a combination of a computer processor, computer memory, and input/output peripheral devices, or microcontroller, that has a dedicated function and comprising at least one processing unit 7, for example a real-time computing function, usually within a larger mechanical or electronic system. By mobile platform it is meant for example a mobile device such as a smartphone or tablet.

Figure 8 demonstrates an example of an embedded prototyping board running an implementation of the proposed method. In this example, the use of the Direnjie framework, (see, for example, Milan Lazarević and Miljan Vuletic. Direnjie: Fast and efficient AI pipeline creation framework for the edge. https://www.darwinedge.com/tbd. Online; accessed: 13-May-2024) demonstrated an example of a rapid and efficient implementation-in this example in native C++ code-of the heart rate detection and tracking pipeline according to the present disclosure.

According to this example implementation of the pipeline, the pipeline comprises a plurality of nodes, for example four nodes creating the pipeline graph: (1) preprocessing node, resizing the input image, for example by detecting a bounding box (BB) in the input frames; (2) inference node, for example performing the face detection inference; (3) tile reduction node, performing the signal extraction; and (4) vital signs estimation node, performing the frequency analysis, heart rate detection and tracking.

Stand-alone applications targeted for several different platforms, (for example, Synaptics Astra, Jetson Orin, or an Apple iPhone as a common consumer platform) may, for example, integrate the built libraries comprising the pipeline and/or comprising the method steps according to the present disclosure, for example the method step S9 for an embedded platform or the steps S5, S7, and S9 for a mobile platform such as an Apple iPhone. Typical configuration settings for, for example, a 30 frames-per-second processing sampling frequency may use for example a window size of 90-150 samples and for example a window slide from 30-60 samples, providing meaningful readings for example starting from a 3-seconds latency after the first iteration of the algorithm, with exact latency depending on lighting or movement noise present in the surrounding environment.

In addition, in an exemplary embodiment of the invention, less than 30 ms execution time of one iteration of the algorithm on embedded chips with limited computational resources such as Synaptics Astra 1640-1680, shown in Fig. 8, may be achieved. Updates of heart rate readings may be produced each second while processing at a 30 frames-per-second rate. Having in mind that the algorithm can run on frame rates as low as 10 frames-per-second, this particular implementation shows the potential of running the algorithm successfully on chips with even lower computational capacities.

The method and system according to the present invention have demonstrated a novel and robust approach for remote nonintrusive heart rate detection and tracking from human skin, applicable to, for example, embedded, mobile, and edge devices. The proposed method employs additional channel creation and preferably penalty-and-award-based detection and tracking for coping with in-band noise disturbances.

The system implemented and deployed to several target embedded platforms has proven the feasibility and robustness of the proposed approach. Steps have also been undertaken for validating the proposed method within clinical and hospital settings, with 95% accuracy of the estimated heart rate observed.

The method and system according to the present invention provide a combination of high accuracy (95%), low latency at algorithm start-up (as low as 3 seconds), fast run-time (30 ms execution time of one algorithm iteration), and resistance to environmental noise ensure for robust and efficient, non-invasive heart-rate detection and tracking.

## <u>REFERENCES</u>

[1] Junyung Park, Hyeon Seok Seok, Sang-Su Kim, and Hangsik Shin. Photoplethysmogram analysis and applications: An integrative review. Frontiers in Physiology, 12, 2022.

[2] Wikipedia. Photoplethysmogram. https://en.wikipedia.org/wiki/Photoplethysmogram, 2024. Online; accessed 13-May-2024.

[3] Wenjin Wang, Albertus C. den Brinker, Sander Stuijk, and Gerard de Haan. Algorithmic principles of remote PPG. IEEE Transactions on Biomedical Engineering, 64(7):1479-1491, 2017.

[4] Daniel McDuff. Camera measurement of physiological vital signs. ACM Comput. Surv., 55(9), jan 2023.

[5] Pireh Pirzada, Adriana Wilde, Gayle H Doherty, and David Harris-Birtill. Remote photoplethysmography (rPPG): A state-of-the-art review. medRxiv, 2023.

[6] TingWu, Vladimir Blazek, and Hans Schmitt. Photoplethysmography imaging: a new noninvasive and noncontact method for mapping of the dermal perfusion changes. November 2000.

[7] Magdalena Lewandowska, Jacek Rumiriski, Tomasz Kocejko, and Jedrzej Nowak. Measuring pulse rate with a webcam - a non-contact method for evaluating cardiac activity. 2011 Federated Conference on Computer Science and Information Systems (FedCSIS), pages 405-10, 2011.

[8] Ming-Zher Poh, Daniel J McDuff, and Rosalind W Picard. Advancements in noncontact, multiparameter physiological measurements using a webcam. IEEE Transactions on Biomedical Engineering, 58(1):7-11, 2010.

[9] Ming-Zher Poh, Daniel J McDuff, and Rosalind W Picard. Non-contact, automated cardiac pulse measurements using video imaging and blind source separation. Optics express, 18(10):10762-74, 2010.

[10] Wenjin Wang, Sander Stuijk, and Gerard De Haan. Exploiting spatial redundancy of image sensor for motion robust rPPG. IEEE Transactions on Biomedical Engineering, 62(2):415-25, 2014.

[11] Gerard de Haan and Vincent Jeanne. Robust pulse rate from chrominance-based rPPG. IEEE Transactions on Biomedical Engineering, 60(10):2878-86, 2013.

[12] Weixuan Chen and Daniel McDuff. Deepphys: Video-based physiological measurement using convolutional attention networks, 2018.

[13] Xin Liu, Josh Fromm, Shwetak Patel, and Daniel McDuff. Multi-task temporal shift attention networks for on-device contactless vitals measurement. arXiv preprint arXiv:2006.03790, 2020.

[14] Zitong Yu, Xiaobai Li, and Guoying Zhao. Remote photoplethysmograph signal measurement from facial videos using spatio-temporal networks, 2019.

[15] Jiankang Deng, Jia Guo, Evangelos Ververas, Irene Kotsia, and Stefanos Zafeiriou. Retinaface: Single-shot multi-level face localisation in the wild. In 2020 IEEE/CVF Conference on Computer Vision and Pattern Recognition (CVPR), pages 5202-5211, 2020.

[16] Yanjia Zhu, Hongxiang Cai, Shuhan Zhang, Chenhao Wang, and Yichao Xiong. Tinaface: Strong but simple baseline for face detection, 2021.

[17] PainChek. The PainChek pain assessment tool. https://www.painchek.com, 2018. Online; accessed 14-May-2024.

[18] nVISO. nVISO launches emotion advisor. https://www.startupticker.ch/en/news/nvisolaunches-new-fintech-solution-to-detect-investor-financial-feelings-about, 2016. Online; accessed 14-May-2024.

[19] Uroš Milosević, Miroslav Markovic, Miljan Vuletić, and Daniel Kämpf. Improving efficiency of retinaface models by controlled branch pruning. In *2023 31st Telecommunications Forum (TELFOR),* pages 1-4, 2023.

[20] Wei Wu, Hanyang Peng, and Shiqi Yu. Yunet: A tiny millisecond-level face detector. Machine Intelligence Research, 20(5):656-65, 2023.

[21] Jemin Lee, Misun Yu, Yongin Kwon, and Taeho Kim. Quantune: Post-training quantization of convolutional neural networks using extreme gradient boosting for fast deployment. Future Generation Computer Systems, 132:124-35, 2022.

[22] NXP. i.MX application processors. https://www.nxp.com/products/processorsandmicrocontrollers/arm-procesors/i-mx-applications-processors. Online; accessed: 14-May-2024.

[23] Synaptics. Synaptics Astra embedded processors. https://www.synaptics.com/products/embeddedprocessors. Online; accessed: 13-May-2024.

[24] John G. Proakis and Dimitris K Manolakis. Digital Signal Processing: Principles, Algorithms and Applications. Prentice Hall, 4 edition, 2006.

[25] Richard G. Lyons. Understanding Digital Signal Processing. Pearson Education, 2010.

[26] James W. Cooley and John W. Tukey. An algorithm for the machine calculation of complex fourier series. Mathematics of Computation, 19:297-301, 1965.

[27] Milan Lazarević and Miljan Vuletić. Direnjie: Fast and efficient AI pipeline creation framework for the edge. https://www.darwinedge.com/tbd. Online; accessed: 13-May-2024.

[28] NVIDIA. Jetson AGX Orin server-class AI performance at the edge. https://www.nvidia.com/enil/autonomous-machines/embedded-systems/jetson-orin. Online; accessed: 13-May-2024.

[29] Wikipedia. Short-time Fourier transform. https://en.wikipedia.org/wiki/Short-time Fourier transform. Online;

accessed 24-September-2024.

[30] Wikipedia.Projection(linearalgebra).https://en.wikipedia.org/wiki/Projection_(linear_alg ebra). Online; accessed 25-September-2024.

[31] Wikipedia. Rotation matrix. https://en.wikipedia.org/wiki/Rotation_matrix. Online; accessed 25-September-2024.

[32] Wikipedia.Fast Fourier transform. https://en.wikipedia.org/wiki/Fast_Fourier_transform. Online; accessed 25-September-2024.

[33] Wikipedia.DiscreteFouriertransform.https://en.wikipedia.org/wiki/Discrete_Fourier_tran sform. Online; accessed 25-September 2024.

**Claims**

1. A method of determining a heart rate (HR) of a human subject (1), comprising:

   - illuminating at least one region of interest (2) of the human subject (1) with a light source (3), the light source (3) being at a distance from the human subject (1);
   - receiving airborne light (4) reflected from the at least one region of interest (2);
   - generating an input colour video signal (5) from the received light (4), and,
   - processing the input colour video signal (5) and estimating the heart rate (HR) from the input colour video signal (5) by

     ◦ (i) generating at least three intermediate signals (IS) from a portion of the input colour video signal (5) generated from the received airborne light (4) reflected from the at least one region of interest (2);
     ◦ (ii) projecting the at least three intermediate signals (IS) onto a two-dimensional skin-colour plane to create two skin-colour plane signals (SCS);
     ◦ (iii) generating a plurality of remote photoplethysmography signals from the two skin-colour plane signals (SCS);
     ◦ (iv) estimating a heart rate value ($H_{\beta i}$) for each remote photoplethysmography signal ($rPPG_i$) based on a frequency content of each remote photoplethysmography signal, and,
     ◦ (v) determining the heart rate (HR) of the human subject (1) based on the estimated heart rate values (Hbr) for each remote photoplethysmography signal ($rPPG_i$); or based on the estimated heart rate values (Hbr) for each remote photoplethysmography signal ($rPPG_i$) and a previously determined heart rate (pHR), determined using the preceding method steps.

2. The method of claim 1, wherein generating each remote photoplethysmography signal ($rPPG_i$) comprises rotating the two skin-colour plane signals (SCS) by a rotation angle ($\beta_i$), and combining the two rotated skin-colour plane signals according to a combination rule, wherein each generated remote photoplethysmography ($rPPG_i$) corresponds to a rotation by the rotation angle ($\beta_i$).

3. The method according to any one of the previous claims, wherein projecting the at least three intermediate signals (IS) onto a two-dimensional skin-colour plane comprises multiplying the at least three intermediate signals (IS) by a projection matrix (P).

4. The method according to claim 2 or 3, wherein rotating the two skin-colour plane signals by the rotation angle ($\beta_i$) comprises multiplying the two skin-colour plane signals (SCS) by a rotation matrix ($R_{\beta i}$) defined by the rotation angle ($\beta_i$).

5. The method according to any one of the previous claims 2 to 4, wherein a plurality of rotation angles comprises a range from 0° and 180°, and wherein each generated remote photoplethysmography signal ($rPPG_i$) corresponds to a rotation by one of the rotation angles ($\beta_i$) in the range.

6. The method according any one of the previous claims, wherein the region of interest (2) comprises a face (3) of the human subject (1).

7. The method according to the previous claim, wherein generating at least three intermediate signals from a portion of the input colour video signal (5) generated from the received light reflected from the at least one the region of interest (2) further comprises detecting the face (3) in a portion of the input colour video signal (5).

8. The method according to any one of the previous claims, wherein determining the heart rate (HR) of the human subject (1) based on the estimated heart rate values (Hbr) for each remote photoplethysmography signal (rPPG$_i$) comprises:

   ◦ (i) grouping the estimated heart rate values (Hbr) for each remote photoplethysmography signal (rPPG$_i$) into at least two clusters;
   ◦ (ii) characterizing each cluster in terms of an average value and a count value, wherein the average value is calculated as the average of all the estimated heart rate values belonging to the cluster, and wherein the count value is found by counting the number of estimated heart rate values belonging to the cluster;
   ◦ (iii) for each cluster, penalizing or reducing its count value if its average heart rate value falls within a user-defined limit (HeartRateInterval) starting at the lower edge (LowHbr), or ending at the upper edge (HighHbr), of a range of heart rate values of physiological interest;
   ◦ (iv) calculating the heart rate (HR) as the average value of the cluster with the greatest count after penalizing the counts.

9. The method according to any one of the previous claims 1 to 7, wherein determining the heart rate of the human subject (1) based on the estimated heart rate values (Hbr) for each signal in the plurality of remote photoplethysmography signals and a previously estimated heart rate (pHR) comprises:

   ◦ (i) grouping the estimated heart rate values (Hbr) for each remote photoplethysmography signal into at least two clusters;
   ◦ (ii) characterizing each cluster in terms of an average value and a count value, wherein the average value is calculated as the average of all the estimated heart rate values belonging to the cluster, and wherein the count value is found by counting the number of estimated heart rate values belonging to the cluster;
   ◦ (iii) for each cluster, either, penalizing or reducing its count value if its average heart rate value falls within a user-defined limit (HeartRateInterval) starting at the lower edge (LowHbr), or ending at the upper edge (HighHbr), of a range of heart rate values of physiological interest, or, awarding or increasing its count value if its average value is less than a user-defined absolute difference (HeartRateInterval/2) from the previously estimated heart rate (pHR);
   ◦ (iv) selecting an intermediate heart rate estimate (NextHbrPick) as the average value of the cluster with the greatest count after penalizing and awarding the counts;
   ◦ (v) calculating the heart rate (HR) by applying an exponential smoothing filter to the intermediate heart rate estimate (NextHbrPick) and a previously estimated heart rate (pHR).

10. The method according to any one of the previous claims, wherein the two-dimension skin-colour plane represents at least one combination of colours of the at least one region of interest (2).

11. A human heart-rate determination system (10), comprising:

   - a camera unit (6) configured to receive airborne light (4) reflected from at least one region of interest (2) of a human subject (1) and configured to generate an input colour video signal (5) from the received airborne light (4), the camera unit (6) being at a distance from the human subject (1);
   - at least one processing unit (7), comprising processing circuitry, individually and/or collectively configured to process the input colour video signal (5) and estimate a heart rate (HR) of the human subject (1) by the use of remote photoplethysmography, wherein the processing unit (7) is configured to

   ◦ (i) generate at least three intermediate signals (IS) from a portion of the input colour video signal (5) comprising the region of interest (2);
   ◦ (ii) project the at least three intermediate signals (IS) onto a two-dimensional skin-colour plane to create two skin-colour plane signals (SCS);
   ◦ (iii) generating a plurality of remote photoplethysmography signals from the two skin-colour plane signals (SCS);
   ◦ (iv) estimate a heart rate value ($H_{\beta i}$) for each remote photoplethysmography signal (rPPG$_i$) in the plurality of remote photoplethysmography signals based on a frequency content of each remote photoplethysmography signal, and,
   ◦ (v) determine the heart rate (HR) of the human subject (1) based on the estimated heart rate values (Hbr) for each remote photoplethysmography signal (rPPG$_i$); or based on the estimated heart rate values (Hbr) for each remote photoplethysmography signal (rPPG$_i$) and a previously estimated heart rate (pHR) according to the preceding steps.

12. The system (10) according to the previous claim, further comprising a light source (3), wherein the light source is at a distance from the human subject (1).

13. The system (10) according to claim 11 or 12, wherein the system comprises or consists of a smartphone, and the smartphone includes the camera unit (6) and the processing unit (7).

14. The system according to claim 11 or 12, wherein the system comprises or consists of a personal computer, and the personal computer includes the camera unit (6) and the processing unit (7).

15. The system (10) according to claim 11 or 12, wherein an embedded device such as any one of an NVIDIA Jetson Orin, Synaptics Astra, or Raspberry Pi comprises the processing unit (7).

**FIGURE 1**

**(PRIOR ART)**

**FIGURE 2**

**FIGURE 3**

**FIGURE 4A**

**FIGURE 4B**

FIGURE 5A

FIGURE 5B

FIGURE 6A

FIGURE 6B

FIGURE 7

FIGURE 8

**5**

S2

IS

S4

SCS

S6

$rPPG_1, \ldots, rPPG_i, \ldots, rPPG_n$

S8

Hbr          Hbr          pHR

S10a                    S10b

HR                      HR

FIGURE 9

S3

4

S5

4

S7

5

5

S2 — IS

S6 — S9

$rPPG_1,...,$
$rPPG_l,...,rPPG_n$

S4

S8

SCS

Hbr

Hbr pHR

S10a

S10b

HR

HR

**FIGURE 10**

| | Europäisches Patentamt European Patent Office Office européen des brevets | **EUROPEAN SEARCH REPORT** | **Application Number** EP 24 21 7581 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TRAN QUOC-VIET ET AL: "Color Distortion Removal for Heart Rate Monitoring in Fitness Scenario", 2019 20TH INTERNATIONAL CONFERENCE ON PARALLEL AND DISTRIBUTED COMPUTING, APPLICATIONS AND TECHNOLOGIES (PDCAT), IEEE, 5 December 2019 (2019-12-05), pages 369-374, XP033738819, DOI: 10.1109/PDCAT46702.2019.00073 [retrieved on 2020-03-09] * abstract * * figure 2 * ----- | 1-15 | INV. A61B5/00 A61B5/024 |
| A | WANG WENJIN ET AL: "Algorithmic Principles of Remote PPG", IEEE TRANSACTIONS ON BIOMEDICAL ENGINEERING, IEEE, USA, vol. 64, no. 7, 1 July 2017 (2017-07-01), pages 1479-1491, XP011653906, ISSN: 0018-9294, DOI: 10.1109/TBME.2016.2609282 [retrieved on 2017-06-15] * abstract * * figure 2 * ----- | 1-15 | |
| | | | TECHNICAL FIELDS SEARCHED (IPC) A61B |
| A | US 10 349 900 B2 (KONINKLIJKE PHILIPS NV [NL]) 16 July 2019 (2019-07-16) * the whole document * ----- -/-- | 1-15 | |

The present search report has been drawn up for all claims

| Place of search | Date of completion of the search | Examiner |
|---|---|---|
| The Hague | 16 May 2025 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| Europäisches Patentamt<br>European Patent Office<br>Office européen des brevets | **EUROPEAN SEARCH REPORT** | Application Number<br>**EP 24 21 7581** |
| --- | --- | --- |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION  (IPC) |
| --- | --- | --- | --- |
| A | MING-ZHER POH ET AL: "Advancements in Noncontact, Multiparameter Physiological Measurements Using a Webcam",<br>IEEE TRANSACTIONS ON BIOMEDICAL ENGINEERING, IEEE, USA,<br>vol. 58, no. 1,<br>1 January 2011 (2011-01-01), pages 7-11,<br>XP011372860,<br>ISSN: 0018-9294, DOI:<br>10.1109/TBME.2010.2086456<br>* the whole document *<br>----- | 1-15 | |
| A | PANIGRAHI ARPITA ET AL: "Contactless HR Measurement from Facial Videos Using Alternative Color Spaces with CEEMDAN",<br>2023 9TH INTERNATIONAL CONFERENCE ON SIGNAL PROCESSING AND COMMUNICATION (ICSC), IEEE,<br>21 December 2023 (2023-12-21), pages 522-527, XP034556895,<br>DOI: 10.1109/ICSC60394.2023.10441625<br>[retrieved on 2024-02-26]<br>* abstract; figure 1 *<br>----- | 1-15 | TECHNICAL FIELDS<br>SEARCHED      (IPC) |

The present search report has been drawn up for all claims

| Place of search | Date of completion of the search | Examiner |
| --- | --- | --- |
| The Hague | 16 May 2025 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 21 7581

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | | Publication date | Patent family member(s) | Publication date |
|---|---|---|---|---|
| US 10349900 | B2 | 16-07-2019 | BR 112015031882 A2 | 25-07-2017 |
| | | | CN 105451646 A | 30-03-2016 |
| | | | EP 2988662 A1 | 02-03-2016 |
| | | | ES 2604812 T3 | 09-03-2017 |
| | | | JP 6114469 B2 | 12-04-2017 |
| | | | JP 2016526947 A | 08-09-2016 |
| | | | RU 2015156254 A | 04-07-2017 |
| | | | US 2015320363 A1 | 12-11-2015 |
| | | | WO 2015169634 A1 | 12-11-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PARK, JUNYUNG et al.** Photoplethysmogram analysis and applications: an integrative review.. *Frontiers in Physiology*, 2022, vol. 12, 808451
- **WENJIN WANG** ; **ALBERTUS C. DEN BRINKER** ; **SANDER STUIJK** ; **GERARD DE HAAN**. Algorithmic principles of remote PPG. *IEEE Transactions on Biomedical Engineering,*, 2017, vol. 64 (7), 1479-1491
- **MCDUFF, DANIEL**. Camera measurement of physiological vital signs.. *ACM Computing Surveys*, 2023, vol. 55 (9), 1-40
- **PIREH PIRZADA et al.** Remote photoplethysmography (rPPG): A state-of-the-art review. *medRxiv,*, 2023
- **WANG, WENJIN et al.** Algorithmic principles of remote PPG.. *IEEE Transactions on Biomedical Engineering*, 2016, vol. 64 (7), 1479-1491
- **CHEN, WEIXUAN** ; **DANIEL MCDUFF**. Deepphys: Video-based physiological measurement using convolutional attention networks.. *Proceedings of the European conference on computer vision (ECCV).*, 2018
- **POH, MING-ZHER** ; **DANIEL J. MCDUFF** ; **ROSALIND W. PICARD**. Advancements in noncontact, multiparameter physiological measurements using a webcam.. *IEEE transactions on biomedical engineering*, 2010, vol. 58 (1), 7-11
- **DE HAAN, GERARD** ; **VINCENT JEANNE**. Robust pulse rate from chrominance-based rPPG.. *IEEE transactions on biomedical engineering*, 2013, vol. 60 (10), 2878-2886
- **DE HAAN, GERARD** ; **VINCENT JEANNE.** Robust pulse rate from chrominance-based rPPG. *IEEE transactions on biomedical engineering*, 2013, vol. 60 (10), 2878-2886
- **DENG, JIANKANG et al.** Retinaface: Single-shot multi-level face localisation in the wild.. *Proceedings of the IEEE/CVF conference on computer vision and pattern recognition*, 2020
- Improving Efficiency of RetinaFace Models by Controlled Branch Pruning.. **MILOSEVIC, UROS et al.** 2023 31st Telecommunications Forum (TELFOR). IEEE, 2023
- **LEE, JEMIN et al.** Quantune: Post-training quantization of convolutional neural networks using extreme gradient boosting for fast deployment.. *Future Generation Computer Systems*, 2022, vol. 132, 124-135
- *NXP. i.MX application processors*, https://www.nxp.com/products/processors andmicrocontrollers/arm-processors/i-mx-applications-processors
- **WENJIN WANG**. Algorithmic principles of remote PPG.. *IEEE Transactions on Biomedical Engineering*, 2017, vol. 64 (7), 1479-1491
- **WENJIN WANG** ; **ALBERTUS C. DEN BRINKER** ; **SANDER STUIJK** ; **GERARD DE HAAN**. Algorithmic principles of remote PPG. *IEEE Transactions on Biomedical Engineering*, 2017, vol. 64 (7), 1479-1491
- **JUNYUNG PARK** ; **HYEON SEOK SEOK** ; **SANG-SU KIM** ; **HANGSIK SHIN**. Photoplethysmogram analysis and applications: An integrative review. *Frontiers in Physiology*, 2022, vol. 12
- Photoplethysmogram. *Wikipedia*, 13 May 2024, https://en.wikipedia.org/wiki/Photoplethysmogram
- **DANIEL MCDUFF**. Camera measurement of physiological vital signs.. *ACM Comput. Surv.*, January 2023, vol. 55 (9)
- **PIREH PIRZADA** ; **ADRIANA WILDE** ; **GAYLE H DOHERTY** ; **DAVID HARRIS-BIRTILL**. Remote photoplethysmography (rPPG): A state-of-the-art review. *medRxiv*, 2023
- **TINGWU** ; **VLADIMIR BLAZEK** ; **HANS SCHMITT**. *Photoplethysmography imaging: a new noninvasive and noncontact method for mapping of the dermal perfusion changes*, November 2000
- **MAGDALENA LEWANDOWSKA** ; **JACEK RUMIR-ISKI** ; **TOMASZ KOCEJKO** ; **JEDRZEJ NOWAK**. Measuring pulse rate with a webcam - a non-contact method for evaluating cardiac activity. *2011 Federated Conference on Computer Science and Information Systems (FedCSIS)*, 2011, 405-10
- **MING-ZHER POH** ; **DANIEL J MCDUFF** ; **ROSALIND W PICARD**. Advancements in noncontact, multiparameter physiological measurements using a webcam. *IEEE Transactions on Biomedical Engineering*, 2010, vol. 58 (1), 7-11
- **MING-ZHER POH** ; **DANIEL J MCDUFF** ; **ROSALIND W PICARD**. Non-contact, automated cardiac pulse measurements using video imaging and blind source separation. *Optics express*, 2010, vol. 18 (10), 10762-74

- **WENJIN WANG** ; **SANDER STUIJK** ; **GERARD DE HAAN**. Exploiting spatial redundancy of image sensor for motion robust rPPG. *IEEE Transactions on Biomedical Engineering*, 2014, vol. 62 (2), 415-25
- **GERARD DE HAAN** ; **VINCENT JEANNE**. Robust pulse rate from chrominance-based rPPG.. *IEEE Transactions on Biomedical Engineering,*, 2013, vol. 60 (10), 2878-86
- **WEIXUAN CHEN** ; **DANIEL MCDUFF**. *Deepphys: Video-based physiological measurement using convolutional attention networks*, 2018
- **XIN LIU** ; **JOSH FROMM** ; **SHWETAK PATEL** ; **DANIEL MCDUFF**. Multi-task temporal shift attention networks for on-device contactless vitals measurement. *arXiv:2006.03790*, 2020
- **ZITONG YU** ; **XIAOBAI LI** ; **GUOYING ZHAO**. *Remote photoplethysmograph signal measurement from facial videos using spatio-temporal networks*, 2019
- **JIANKANG DENG** ; **JIA GUO** ; **EVANGELOS VERVERAS** ; **IRENE KOTSIA** ; **STEFANOS ZAFEIRIOU**. Retinaface: Single-shot multi-level face localisation in the wild. *2020 IEEE/CVF Conference on Computer Vision and Pattern Recognition (CVPR)*, 2020, 5202-5211
- **YANJIA ZHU** ; **HONGXIANG CAI** ; **SHUHAN ZHANG** ; **CHENHAO WANG** ; **YICHAO XIONG**. *Tinaface: Strong but simple baseline for face detection*, 2021
- The PainChek pain assessment tool.. *PainChek*, 2018, https://www.painchek.com
- nVISO launches emotion advisor. *nVISO*, 2016, https://www.startupticker.ch/en/news/nvisolaunches-new-fintech-solution-to-detect investor-financial-feelings-about
- **WEI WU** ; **HANYANG PENG** ; **SHIQI YU**. Yunet: A tiny millisecond-level face detector. *Machine Intelligence Research,*, 2023, vol. 20 (5), 656-65
- **JEMIN LEE** ; **MISUN YU** ; **YONGIN KWON** ; **TAEHO KIM**. Quantune: Post-training quantization of convolutional neural networks using extreme gradient boosting for fast deployment. *Future Generation Computer Systems*, 2022, vol. 132, 124-35
- i.MX application processors. https://www.nxp.com/products/processorsandmicrocontrollers/arm-processors/i-mx-applications-processors. *NXP*, 14 May 2024
- Synaptics Astra embedded processors. *Synaptics*, 13 May 2024, https://www.synaptics.com/products/embeddedprocessors
- **JOHN G. PROAKIS** ; **DIMITRIS K MANOLAKIS**. Digital Signal Processing: Principles, Algorithms and Applications. Prentice Hall, 2006
- **RICHARD G. LYONS**. Understanding Digital Signal Processing. Pearson Education, 2010
- **JAMES W. COOLEY** ; **JOHN W. TUKEY**. An algorithm for the machine calculation of complex fourier series. *Mathematics of Computation,*, 1965, vol. 19, 297-301
- Jetson AGX Orin server-class AI performance at the edge. *NVIDIA*, 13 May 2024, https://www.nvidia.com/enil/autonomous-machines/embedded-systems/jetson-orin
- Short-time Fourier transform. *Wikipedia*, 24 September 2024, https://en.wikipedia.org/wiki/Short-time Fourier transform
- Projection(linearalgebra). *Wikipedia*, 25 September 2024, https://en.wikipedia.org/wiki/Projection_(linear_alg ebra)
- Rotation matrix. *Wikipedia*, 25 September 2024, https://en.wikipedia.org/wiki/Rotation_matrix
- Fast Fourier transform. *Wikipedia*, 25 September 2024, https://en.wikipedia.org/wiki/Fast_Fourier_transform
- DiscreteFouriertransform. *Wikipedia*, 25 September 2024, https://en.wikipedia.org/wiki/Discrete_Fourier_tran sform